# EUROPEAN PATENT SPECIFICATION

(11) **EP 1 311 242 B1**
(45) Date of publication and mention of the grant of the patent: **31.03.2010**
(21) Application number: 01966056.2
(22) Date of filing: 22.08.2001
(51) Int. Cl.: A61K 9/14, A61K 9/22, A61K 9/50, A61K 47/42, C07K 1/02, C07K 1/13

(54) **ACTIVE AGENT DELIVERY SYSTEMS AND METHODS FOR PROTECTING AND ADMINISTERING ACTIVE AGENTS**
DARREICHUNGSSYSTEME VON WIRKSTOFFEN UND METHODEN ZUM SCHUTZ UND ZUR VERABREICHUNG VON WIRKSTOFFEN
SYSTEMES DE LIBERATIONS D'AGENTS ACTIFS ET PROCEDES DE PROTECTION ET D'ADMINISTRATION D'AGENTS ACTIFS

(30) Priority: 22.08.2000 US 642820; 14.11.2000 US 247622 P; 14.11.2000 US 247621 P; 14.11.2000 US 247620 P; 14.11.2000 US 247595 P; 14.11.2000 US 247594 P; 14.11.2000 US 247635 P; 14.11.2000 US 247634 P; 14.11.2000 US 247606 P; 14.11.2000 US 247607 P; 14.11.2000 US 247608 P; 14.11.2000 US 247609 P; 14.11.2000 US 247610 P; 14.11.2000 US 247611 P; 14.11.2000 US 247702 P; 14.11.2000 US 247701 P; 14.11.2000 US 247700 P; 14.11.2000 US 247699 P; 14.11.2000 US 247698 P; 14.11.2000 US 247807 P; 14.11.2000 US 247833 P; 14.11.2000 US 247832 P; 14.11.2000 US 247927 P; 14.11.2000 US 247926 P; 14.11.2000 US 247930 P; 14.11.2000 US 247929 P; 14.11.2000 US 247928 P; 14.11.2000 US 247797 P; 14.11.2000 US 247805 P; 14.11.2000 US 247804 P; 14.11.2000 US 247803 P; 14.11.2000 US 247802 P; 14.11.2000 US 247801 P; 14.11.2000 US 247800 P; 14.11.2000 US 247799 P; 14.11.2000 US 247798 P; 14.11.2000 US 247561 P; 14.11.2000 US 247560 P; 14.11.2000 US 247559 P; 14.11.2000 US 247558 P; 14.11.2000 US 247556 P; 14.11.2000 US 247612 P; 14.11.2000 US 247613 P; 14.11.2000 US 247614 P; 14.11.2000 US 247615 P; 14.11.2000 US 247616 P; 14.11.2000 US 247617 P; 14.11.2000 US 247633 P; 14.11.2000 US 247632 P; 14.11.2000 US 247631 P; 14.11.2000 US 247630 P; 14.11.2000 US 247629 P; 14.11.2000 US 247628 P; 14.11.2000 US 247627 P; 14.11.2000 US 247626 P; 14.11.2000 US 247625 P; 14.11.2000 US 247624 P; 14.11.2000 US 247806 P; 14.11.2000 US 247563 P; 14.11.2000 US 247795 P; 14.11.2000 US 247794 P; 14.11.2000 US 247793 P; 14.11.2000 US 247792 P; 14.11.2000 US 247791 P; 14.11.2000 US 247790 P; 14.11.2000 US 247789 P; 14.11.2000 US 247788 P; 14.11.2000 US 247787 P; 14.11.2000 US 247786 P; 14.11.2000 US 247785 P; 14.11.2000 US 247784 P; 14.11.2000 US 247783 P; 14.11.2000 US 247782 P; 14.11.2000 US 247781 P; 14.11.2000 US 247780 P; 14.11.2000 US 247779 P; 14.11.2000 US 247778 P; 14.11.2000 US 247777 P; 14.11.2000 US 247776 P; 14.11.2000 US 247775 P; 14.11.2000 US 247774 P; 14.11.2000 US 247773 P; 14.11.2000 US 247772 P; 14.11.2000 US 247770 P; 14.11.2000 US 247769 P; 14.11.2000 US 247768 P; 14.11.2000 US 247767 P; 14.11.2000 US 247766 P; 14.11.2000 US 247871 P; 14.11.2000 US 247872 P; 14.11.2000 US 247873 P; 14.11.2000 US 247874 P; 14.11.2000 US 247875 P; 14.11.2000 US 247981 P; 14.11.2000 US 247982 P; 14.11.2000 US 247983 P; 14.11.2000 US 247984 P; 14.11.2000 US 247745 P; 14.11.2000 US 247744 P; 14.11.2000 US 247743 P; 14.11.2000 US 247742 P; 14.11.2000 US 247623 P; 14.11.2000 US 247985 P; 14.11.2000 US 247840 P; 14.11.2000 US 247839 P; 14.11.2000 US 247838 P; 14.11.2000 US 247837 P; 14.11.2000 US 247836 P; 14.11.2000 US 247889 P; 14.11.2000 US 247890 P; 14.11.2000 US 247891 P; 14.11.2000 US 247892 P; 14.11.2000 US 247893 P; 14.11.2000 US 247741 P; 14.11.2000 US 247740 P; 14.11.2000 US 247739 P; 14.11.2000 US 247738 P; 14.11.2000 US 247737 P; 14.11.2000 US 247736 P; 14.11.2000 US 247735 P; 14.11.2000 US 247734 P; 14.11.2000 US 247733 P; 14.11.2000 US 247732 P; 14.11.2000 US 247731 P; 14.11.2000 US 247730 P; 14.11.2000 US 247728 P; 14.11.2000 US 247729 P; 14.11.2000 US 247727 P; 14.11.2000 US 247726 P; 14.11.2000 US 247761 P; 14.11.2000 US 247760 P; 14.11.2000 US 247759 P; 14.11.2000 US 247758 P; 14.11.2000 US 247757 P; 14.11.2000 US 247756 P; 14.11.2000 US 247765 P; 14.11.2000 US 247764 P; 14.11.2000 US 247763 P; 14.11.2000 US 247762 P; 14.11.2000 US 247755 P; 14.11.2000 US 247746 P; 14.11.2000 US 247725 P; 14.11.2000 US 247724 P; 14.11.2000 US 247723 P; 14.11.2000 US 247722 P; 14.11.2000 US 247721 P; 14.11.2000 US 247720 P; 14.11.2000 US 247719 P; 14.11.2000 US 247718 P; 14.11.2000 US 247717 P; 14.11.2000 US 247716 P; 14.11.2000 US 247754 P; 14.11.2000 US 247753 P; 14.11.2000 US 247752 P; 14.11.2000 US 247751 P; 14.11.2000 US 247750 P; 14.11.2000 US 247749 P; 14.11.2000 US 247748 P; 14.11.2000 US 247747 P; 14.11.2000 US 247796 P; 14.11.2000 US 247815 P; 14.11.2000 US 247814 P; 14.11.2000 US 247813 P; 14.11.2000 US 247812 P; 14.11.2000 US 247811 P; 14.11.2000 US 247810 P; 14.11.2000 US 247809 P; 14.11.2000 US 247808 P; 14.11.2000 US 247885 P; 14.11.2000 US 247884 P; 14.11.2000 US 247883 P; 14.11.2000 US 247882 P; 14.11.2000 US 247881 P; 14.11.2000 US 247880 P; 14.11.2000 US 247879 P; 14.11.2000 US 247878 P; 14.11.2000 US 247826 P; 14.11.2000 US 247835 P; 14.11.2000 US 247834 P; 14.11.2000 US 247897 P; 14.11.2000 US 247896 P; 14.11.2000 US 247895 P; 14.11.2000 US 247894 P; 14.11.2000 US 247901 P; 14.11.2000 US 247900 P; 14.11.2000 US 247899 P; 14.11.2000 US 247898 P; 14.11.2000 US 247903 P; 14.11.2000 US 247902 P; 14.11.2000 US 247919 P; 14.11.2000 US 247918 P; 14.11.2000 US 247917 P; 14.11.2000 US 247916 P; 14.11.2000 US 247915 P; 14.11.2000 US 247914 P; 14.11.2000 US 247913 P; 14.11.2000 US 247912 P; 14.11.2000 US 247911 P; 14.11.2000 US 247910 P; 14.11.2000 US 247877 P; 14.11.2000 US 247876 P; 14.11.2000 US 247707 P; 14.11.2000 US 247706 P; 14.11.2000 US 247705 P; 14.11.2000 US 247704 P; 14.11.2000 US 247703 P; 14.11.2000 US 247692 P; 14.11.2000 US 247691 P; 14.11.2000 US 247690 P; 14.11.2000 US 247689 P; 14.11.2000 US 247688 P; 14.11.2000 US 247687 P; 14.11.2000 US 247686 P; 14.11.2000 US 247685 P; 14.11.2000 US 247684 P; 14.11.2000 US 247683 P; 14.11.2000 US 247694 P; 14.11.2000 US 247693 P; 14.11.2000 US 247712 P; 14.11.2000 US 247711 P; 14.11.2000 US 247710 P; 14.11.2000 US 247709 P; 14.11.2000 US 247708 P; 14.11.2000 US 247697 P; 14.11.2000 US 247696 P; 14.11.2000 US 247695 P; 14.11.2000 US 247565 P; 14.11.2000 US 247564 P; 14.11.2000 US 247545 P; 14.11.2000 US 247546 P; 14.11.2000 US 247547 P; 14.11.2000 US 247548 P; 14.11.2000 US 247568 P; 14.11.2000 US 247570 P; 14.11.2000 US 247580 P; 14.11.2000 US 247555 P; 14.11.2000 US 247554 P; 14.11.2000 US 247553 P; 14.11.2000 US 247552 P; 14.11.2000 US 247551 P; 14.11.2000 US 247682 P; 14.11.2000 US 247681 P; 14.11.2000 US 247680 P; 14.11.2000 US 247679 P; 14.11.2000 US 247678 P; 14.11.2000 US 247677 P; 14.11.2000 US 247676 P; 14.11.2000 US 247655 P; 14.11.2000 US 247645 P; 14.11.2000 US 247656 P; 14.11.2000 US 247771 P; 16.11.2000 US 248607 P; 16.11.2000 US 248611 P; 16.11.2000 US 248609 P; 16.11.2000 US 248608 P; 16.11.2000 US 248606 P; 16.11.2000 US 248604 P; 16.11.2000 US 248603 P; 16.11.2000 US 248601 P; 16.11.2000 US 248600 P; 16.11.2000 US 248712 P; 16.11.2000 US 248711 P; 16.11.2000 US 248709 P; 16.11.2000 US 248708 P; 16.11.2000 US 248707 P; 16.11.2000 US 248706 P; 16.11.2000 US 248705 P; 16.11.2000 US 248704 P; 16.11.2000 US 248703 P; 16.11.2000 US 248702 P; 16.11.2000 US 248701 P; 16.11.2000 US 248700 P; 16.11.2000 US 248699 P; 16.11.2000 US 248698 P; 16.11.2000 US 248697 P; 16.11.2000 US 248696 P; 16.11.2000 US 248695 P; 16.11.2000 US 248694 P; 16.11.2000 US 248693 P; 16.11.2000 US 248692 P; 16.11.2000 US 248691 P; 16.11.2000 US 248710 P; 16.11.2000 US 248689 P; 16.11.2000 US 248688 P; 16.11.2000 US 248686 P; 16.11.2000 US 248720 P; 16.11.2000 US 248719 P; 16.11.2000 US 248718 P; 16.11.2000 US 248716 P; 16.11.2000 US 248715 P; 16.11.2000 US 248714 P; 16.11.2000 US 248713 P; 16.11.2000 US 248536 P; 16.11.2000 US 248535 P; 16.11.2000 US 248733 P; 16.11.2000 US 248732 P; 16.11.2000 US 248731 P; 16.11.2000 US 248730 P; 16.11.2000 US 248729 P; 16.11.2000 US 248728 P; 16.11.2000 US 248727 P; 16.11.2000 US 248726 P; 16.11.2000 US 248725 P; 16.11.2000 US 248724 P; 16.11.2000 US 248723 P; 16.11.2000 US 248722 P; 16.11.2000 US 248721 P; 16.11.2000 US 248540 P; 16.11.2000 US 248539 P; 16.11.2000 US 248538 P; 16.11.2000 US 248537 P; 16.11.2000 US 248533 P; 16.11.2000 US 248532 P; 16.11.2000 US 248531 P; 16.11.2000 US 248530 P; 16.11.2000 US 248529 P; 16.11.2000 US 248528 P; 16.11.2000 US 248527 P; 16.11.2000 US 248526 P; 16.11.2000 US 248525 P; 16.11.2000 US 248524 P; 16.11.2000 US 248670 P; 16.11.2000 US 248789 P; 16.11.2000 US 248599 P; 16.11.2000 US 248745 P; 16.11.2000 US 248746 P; 16.11.2000 US 248747 P; 16.11.2000 US 248748 P; 16.11.2000 US 248744 P; 16.11.2000 US 248743 P; 16.11.2000 US 248756 P; 16.11.2000 US 248602 P; 16.11.2000 US 248598 P; 16.11.2000 US 248597 P; 16.11.2000 US 248596 P; 16.11.2000 US 248595 P; 16.11.2000 US 248594 P; 16.11.2000 US 248858 P; 16.11.2000 US 248857 P; 16.11.2000 US 248856 P; 16.11.2000 US 248855 P; 16.11.2000 US 248854 P; 16.11.2000 US 248853 P; 16.11.2000 US 248852 P; 16.11.2000 US 248851 P; 16.11.2000 US 248850 P; 16.11.2000 US 248849 P; 16.11.2000 US 248848 P; 16.11.2000 US 248792 P; 16.11.2000 US 248790 P; 16.11.2000 US 248669 P; 16.11.2000 US 248668 P; 16.11.2000 US 248667 P; 16.11.2000 US 248666 P; 16.11.2000 US 248665 P; 16.11.2000 US 248664 P; 16.11.2000 US 248793 P; 16.11.2000 US 248791 P; 16.11.2000 US 248684 P; 16.11.2000 US 248683 P; 16.11.2000 US 248682 P; 16.11.2000 US 248681 P; 16.11.2000 US 248680 P; 16.11.2000 US 248671 P; 16.11.2000 US 248679 P; 16.11.2000 US 248675 P; 16.11.2000 US 248676 P; 16.11.2000 US 248677 P; 16.11.2000 US 248678 P; 16.11.2000 US 248673 P; 16.11.2000 US 248674 P; 16.11.2000 US 248672 P; 16.11.2000 US 248784 P; 16.11.2000 US 248785 P; 16.11.2000 US 248786 P; 16.11.2000 US 248775 P; 16.11.2000 US 248773 P; 16.11.2000 US 248766 P; 16.11.2000 US 248765 P; 16.11.2000 US 248833 P; 16.11.2000 US 248783 P; 16.11.2000 US 248781 P; 16.11.2000 US 248780 P; 16.11.2000 US 248778 P; 16.11.2000 US 248767 P; 16.11.2000 US 248787 P; 16.11.2000 US 248774 P; 16.11.2000 US 248764 P; 16.11.2000 US 248782 P; 16.11.2000 US 248779 P; 16.11.2000 US 248685 P; 16.11.2000 US 248772 P; 16.11.2000 US 248771 P; 16.11.2000 US 248777 P; 16.11.2000 US 248776 P; 16.11.2000 US 248770 P; 16.11.2000 US 248768 P; 16.11.2000 US 248769 P; 16.11.2000 US 248796 P; 16.11.2000 US 248797 P; 16.11.2000 US 248795 P; 16.11.2000 US 248794 P; 16.11.2000 US 248663 P; 16.11.2000 US 248662 P; 16.11.2000 US 248660 P; 16.11.2000 US 248659 P; 16.11.2000 US 248658 P; 16.11.2000 US 248656 P; 16.11.2000 US 248654 P; 16.11.2000 US 248653 P; 16.11.2000 US 248651 P; 16.11.2000 US 248650 P; 16.11.2000 US 248648 P; 16.11.2000 US 248647 P; 16.11.2000 US 248645 P; 16.11.2000 US 248643 P; 16.11.2000 US 248642 P; 16.11.2000 US 248640 P; 16.11.2000 US 248637 P; 16.11.2000 US 248636 P; 16.11.2000 US 248634 P; 16.11.2000 US 248632 P; 16.11.2000 US 248631 P; 16.11.2000 US 248630 P; 16.11.2000 US 248629 P; 16.11.2000 US 248627 P; 16.11.2000 US 248625 P; 16.11.2000 US 248763 P; 16.11.2000 US 248761 P; 16.11.2000 US 248759 P; 16.11.2000 US 248757 P; 16.11.2000 US 248754 P; 16.11.2000 US 248753 P; 16.11.2000 US 248749 P; 16.11.2000 US 248616 P; 16.11.2000 US 248615 P; 16.11.2000 US 248614 P; 16.11.2000 US 248613 P; 16.11.2000 US 248612 P; 16.11.2000 US 248605 P; 16.11.2000 US 248610 P; 16.11.2000 US 248661 P; 16.11.2000 US 248657 P; 16.11.2000 US 248655 P; 16.11.2000 US 248652 P; 16.11.2000 US 248649 P; 16.11.2000 US 248646 P; 16.11.2000 US 248644 P; 16.11.2000 US 248641 P; 16.11.2000 US 248639 P; 16.11.2000 US 248638 P; 16.11.2000 US 248635 P; 16.11.2000 US 248633 P; 16.11.2000 US 248628 P; 16.11.2000 US 248626 P; 16.11.2000 US 248624 P; 16.11.2000 US 248762 P; 16.11.2000 US 248760 P; 16.11.2000 US 248758 P; 16.11.2000 US 248755 P; 16.11.2000 US 248752 P; 16.11.2000 US 248751 P; 16.11.2000 US 248750 P; 16.11.2000 US 248742 P; 16.11.2000 US 248741 P; 16.11.2000 US 248740 P; 16.11.2000 US 248739 P; 16.11.2000 US 248736 P; 16.11.2000 US 248735 P; 16.11.2000 US 248734 P; 16.11.2000 US 248623 P; 16.11.2000 US 248622 P; 16.11.2000 US 248621 P; 16.11.2000 US 248738 P; 16.11.2000 US 248737 P; 16.11.2000 US 248620 P; 16.11.2000 US 248619 P; 16.11.2000 US 248618 P; 16.11.2000 US 248617 P; 16.11.2000 US 248687 P; 16.11.2000 US 248690 P; 16.11.2000 US 248717 P
(43) Date of publication of application: 21.05.2003
(60) Divisional application: 09152598.0
(73) Proprietor: Shire LLC, Florence, KY 41042 (US)
(72) Inventor: PICCARIELLO, Thomas, Blacksburg, VA 24060 (US); OLON, Lawrence, P., Bristol, Tennessee 37620 (US); KIRK, Randall, J., Radford, VA 24060 (US)
(74) Representative: Atkinson, Jonathan David Mark
(86) International application number: PCT/US2001/026142
(87) International publication number: WO 2002/034237

(56) References cited:
- WO-A-99/49901
- WO-A2-97/36616
- US-A- 3 846 399
- US-A- 4 356 166
- US-A- 5 087 616
- US-A- 5 238 714
- US-A- 5 534 496
- US-A- 5 846 743
- US-A- 5 882 645
- US-A- 5 898 033
- US-A- 5 910 569
- US-A- 5 948 750
- US-A- 6 005 004
- US-A- 6 030 941
- MA Y-A ET AL: "Enzymatic mechanism of thyroxine biosynthesis. Identification of the 'lost three-carbon fragment' [14]" JOURNAL OF THE AMERICAN CHEMICAL SOCIETY 29 SEP 1999 UNITED STATES, vol. 121, no. 38, 29 September 1999 (1999-09-29), pages 8967-8968, XP002323008 ISSN: 0002-7863
- SCHMIDT ET AL.: 'Peptide-linked 1,3-dialkyl-3-acyltriazenes: Gastrin receptor directed antineoplastic alkylating agents' JOURNAL OF MEDICINAL CHEMISTRY vol. 37, no. 22, 1994, pages 3812 - 3817, XP002949122

## Description

### Field of the Invention

The present invention relates to active agent delivery systems and, more specifically, to compositions that comprise polypeptides covalently attached to active agents and methods for protecting and administering active agents.

### Background of the Invention

Active agent delivery systems are often critical for the effective delivery of a biologically active agent (active agent) to the appropriate target. The importance of these systems becomes magnified when patient compliance and active agent stability are taken under consideration. For instance, one would expect patient compliance to increase markedly if an active agent is administered orally in lieu of an injection or another invasive technique. Increasing the stability of the active agent, such as prolonging shelf life or survival in the stomach, will assure dosage reproducibility and perhaps even reduce the number of dosages required which could improve patient compliance.

Absorption of an orally administered active agent is often blocked by the harshly acidic stomach milieu, powerful digestive enzymes in the GI tract, permeability of cellular membranes and transport across lipid bilayers. Incorporating adjuvants such as resorcinol, surfactants, polyethylene glycol (PEG) or bile acids enhance permeability of cellular membranes. Microencapsulating active agents using protenoid microspheres, liposomes or polysaccharides have been effective in abating enzyme degradation of the active agent. Enzyme inhibiting adjuvants have also been used to prevent enzyme degradation. Enteric coatings have been used as a protector of pharmaceuticals in the stomach.

Active agent delivery systems also provide the ability to control the release of the active agent. For example, formulating diazepam with a copolymer of glutamic acid and aspartic acid enables a sustained release of the active agent. As another example, copolymers of lactic acid and glutaric acid are used to provide timed release of human growth hormone. A wide range of pharmaceuticals purportedly provide sustained release through microencapsulation of the active agent in amides of dicarboxylic acids, modified amino acids or thermally condensed amino acids. Slow release rendering additives can also be intermixed with a large array of active agents in tablet formulations.

Each of these technologies imparts enhanced stability and time-release properties to active agent substances. Unfortunately, these technologies suffer from several shortcomings. Incorporation of the active agent is often dependent on diffusion into the microencapsulating matrix, which may not be quantitative and may complicate dosage reproducibility. In addition, encapsulated drugs rely on diffusion out of the matrix, which is highly dependant on the water solubility of the active agent. Conversely, water-soluble microspheres swell by an infinite degree and, unfortunately, may release the active agent in bursts with little active agent available for sustained release. Furthermore, in some technologies, control of the degradation process required for active agent release is unreliable. For example, an enterically coated active agent depends on pH to release the active agent and, as such, is difficult to control the rate of release.

May, A. et al. [« Enzymatic Mechanism of Thyroxine Biosynthesis-Identification of the « lost three-carbon fragment » Journal of the American Chemical Society (1989), 121 (38):8967-68] disclose T4 - polylysine conjugates. Copolymers of T3/T4 are disclosed in US-A-5910569.

In the past, use has been made of amino acid side chains of polypeptides as pendant groups to which active agents can be attached. These technologies typically require the use of spacer groups between the amino acid pendant group and the active agent. The peptide-drug conjugates of this class of drug delivery system rely on enzymes in the bloodstream for the release of the drug and, as such, are not used for oral administration. Examples of timed and targeted release of injectable or subcutaneous pharmaceuticals include: linking of norethindrone, via a hydroxypropyl spacer, to the gamma carboxylate of polyglutamic acid; and linking of nitrogen mustard, via a peptide spacer, to the gamma carbamide of polyglutamine. Dexamethasone has been covalently attached directly to the beta carboxylate of polyaspartic acid without a spacer group. This prodrug formulation was designed as a colon-specific drug delivery system where the drug is released by bacterial hydrolytic enzymes residing in the large intestines. The released dexamethasone active agent, in turn, was targeted to treat large bowel disorders and was not intended to be absorbed into the bloodstream. Yet another technology combines the advantages of covalent drug attachment with liposome formation where the active ingredient is attached to highly ordered lipid films (known as HARs) via a peptide linker. Thus, there has been no drug delivery system, heretofore reported, that incorporates the concept of attaching an active ingredient to a polypeptide pendant group with its targeted delivery into the bloodstream via oral administration.

It is also important to control the molecular weight, molecular size and particle size of the active agent delivery system. Variable molecular weights have unpredictable diffusion rates and pharmacokinetics. High molecular weight carriers are digested slowly or late, as in the case of naproxen-linked dextran, which is digested almost exclusively in the colon by bacterial enzymes. High molecular weight microspheres usually have high moisture content which may present a problem with water labile active ingredients. Particle size not only becomes a problem with injectable drugs, as in the HAR application, but absorption through the brush-border membrane of the intestines is limited to less than 5 microns.

### Summary of the Invention

The present invention provides covalent attachment of active agents to a polymer of peptides or amino acids. The invention is distinguished from the above mentioned technologies by virtue of covalently attaching the active agent, which includes, for example, pharmaceutical drugs and nutrients, to the N-terminus, the C-terminus or directly to the amino acid side chain of an oligopeptide or polypeptide, also referred to herein as a carrier peptide. In certain applications, the polypeptide will stabilize the active agent, primarily in the stomach, through conformational protection. In these applications, delivery of the active agent is controlled, in part, by the kinetics of unfolding of the carrier peptide. Upon entry into the upper intestinal tract, indigenous enzymes release the active ingredient for absorption by the body by selectively hydrolyzing the peptide bonds of the carrier peptide. This enzymatic action introduces a second order sustained release mechanism.

The invention provides a composition comprising a polypeptide and an active agent covalently attached to the polypeptide. The polypeptide is a homopolymer of one of the twenty naturally occurring amino acids (L or D isomers), or an isomer, analogue, or derivative thereof.

According to the present invention there is provided a drug-peptide conjugate comprising a polypeptide consisting of polyglutamic acid, and a copolymer of T3 and T4 covalently attached through an alcohol functionality of said copolymer to the N-terminus of said polypeptide.

The active agent preferably is covalently attached to the N-terminus of the polypeptide. The active agent is an alcohol and is covalently attached to the N-terminus of the polypeptide.

The composition of the invention can also include one or more of a microencapsulating agent, an adjuvant and a pharmaceutically acceptable excipient. The microencapsulating agent can be selected from polyethylene glycol (PEG), an amino acid, a sugar and a salt. When an adjuvant is included in the composition, the adjuvant preferably activates an intestinal transporter.

Preferably, the composition of the invention is in the form of an ingestable tablet, an intravenous preparation or an oral suspension. The active agent can be conformationally protected by folding of the polypeptide about the active agent. In another embodiment, the polypeptide is capable of releasing the active agent from the composition in a pH-dependent manner.

The invention also provides a method for protecting an active agent from degradation comprising covalently attaching the active agent to a polypeptide.

The invention also provides a method for controlling release of an active agent from a composition wherein the composition comprises a polypeptide, the method comprising covalently attaching the active agent to the polypeptide.

The invention also provides a method for delivering an active agent to a patient, the patient being a human or a non-human animal, comprising administering to the patient a composition comprising a polypeptide and an active agent covalently attached to the polypeptide. In a preferred embodiment, the active agent is released from the composition by an enzyme-catalyzed release. In another preferred embodiment, the active agent is released in a time-dependent manner based on the pharmacokinetics of the enzyme-catalyzed release. In another preferred embodiment, the composition further comprises a microencapsulating agent and the active agent is released from the composition by dissolution of the microencapsulating agent. In another preferred embodiment, the active agent is released from the composition by a pH-dependent unfolding of the polypeptide. In another preferred embodiment, the active agent is released from the composition in a sustained release. In yet another preferred embodiment, the composition further comprises an adjuvant covalently attached to the polypeptide and release of the adjuvant from the composition is controlled by the polypeptide. The adjuvant can be microencapsulated into a carrier peptide-drug conjugate for biphasic release of active ingredients.

A method for preparing a composition comprising a polypeptide and an active agent covalently attached to the polypeptide comprises the steps of:
(a) attaching the active agent to a side chain of an amino acid to form an active agent/amino acid complex;
(b) forming an active agent/amino acid complex N-carboxyanhydride (NCA) from the active agent/amino acid complex; and
(c) polymerizing the active agent/amino acid complex N-carboxyanhydride (NCA).

In a preferred embodiment, the active agent is a pharmaceutical agent or an adjuvant. In another preferred embodiment, steps (a) and (b) are repeated prior to step (c) with a second active agent. When steps (a) and (b) are repeated prior to step (c) with a second agent, the active agent and second active agent can be copolymerized in step (c). In another preferred embodiment, the amino acid is glutamic acid and the active agent is released from the glutamic acid as a dimer upon a hydrolysis of the polypeptide and wherein the active agent is released from the glutamic acid by coincident intramolecular transamination. In another preferred embodiment, the glutamic acid is replaced by an amino acid selected from the group consisting of aspartic acid, arginine, asparagine, cysteine, lysine, threonine, and serine, and wherein the active agent is attached to the side chain of the amino acid to form an amide, a thioester, an ester, an ether, a urethane, a carbonate, an anhydride or a carbamate. In yet another preferred embodiment, the glutamic acid is replaced by a synthetic amino acid with a pendant group comprising an amine, an alcohol, a sulfhydryl, an amide, a urea, or an acid functionality.

It is to be understood that both the foregoing general description and the following detailed description are exemplary, but are not restrictive, of the invention.

### BRIEF DESCRIPTION OF THE DRAWINGS

The invention is best understood from the following detailed description when read in connection with the accompanying drawing. Included in the drawing are the following figures.
Fig 1 illustrates an alcohol active agent/N-terminus scheme of the invention.

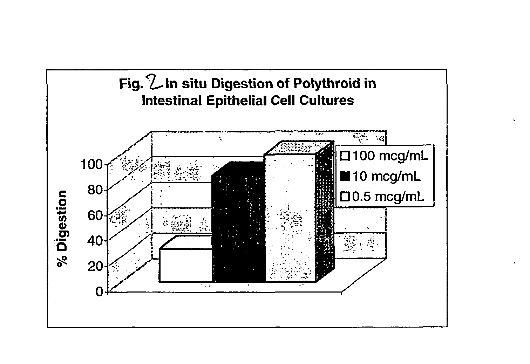

Fig. 2 illustrates the in situ digestion of polythroid in intestinal epithelial cell cultures.
Fig. 3 illlustrates basolateral T4 concentrations.
Fig. 4 illustrates the polythroid concentration of basal versus basolateral.
Fig. 5 illustrates T4 analysis in gastric simulator versus intestinal simulator.
Fig. 6 illustrates T3 analysis in gastric simulator versus intestinal simulator.

### DETAILED DESCRIPTION OF THE INVENTION

The present invention provides several benefits for active agent delivery. First, the invention can stabilize the active agent and prevent digestion in the stomach. In addition, the pharmacologic effect can be prolonged by delayed release of the active agent. Furthermore, active agents can be combined to produce synergistic effects. Also, absorption of the active agent in the intestinal tract can be enhanced. The invention also allows targeted delivery of active agents to specifics sites of action.

Proteins, oligopeptides and polypeptides are polymers of amino acids that have primary, secondary and tertiary structures. The secondary structure of the protein is the local conformation of the polypeptide chain and consists of helices, pleated sheets and turns. The protein's amino acid sequence and the structural constraints on the conformations of the chain determine the spatial arrangement of the molecule. The folding of the secondary structure and the spatial arrangement of the side chains constitute the tertiary structure.

Proteins fold because of the dynamics associated between neighboring atoms on the protein and solvent molecules. The thermodynamics of protein folding and unfolding are defined by the free energy of a particular condition of the protein that relies on a particular model. The process of protein folding involves, amongst other things, amino acid residues packing into a hydrophobic core. The amino acid side chains inside the protein core occupy the same volume as they do in amino acid crystals. The folded protein interior is therefore more like a crystalline solid than an oil drop and so the best model for determining forces contributing to protein stability is the solid reference state.

The major forces contributing to the thermodynamics of protein folding are Van der Waals interactions, hydrogen bonds, electrostatic interactions, configurational entropy and the hydrophobic effect. Considering protein stability, the hydrophobic effect refers to the energetic consequences of removing apolar groups from the protein interior and exposing them to water. Comparing the energy of amino acid hydrolysis with protein unfolding in the solid reference state, the hydrophobic effect is the dominant force. Hydrogen bonds are established during the protein fold process and intramolecular bonds are formed at the expense of hydrogen bonds with water. Water molecules are "pushed out" of the packed, hydrophobic protein core. All of these forces combine and contribute to the overall stability of the folded protein where the degree to which ideal packing occurs determines the degree of relative stability of the protein. The result of maximum packing is to produce a center of residues or hydrophobic core that has maximum shielding from solvent.

Since it is likely that lipophilic drugs would reside in the hydrophobic core of a peptide, it would require energy to unfold the peptide before the drug can be released. The unfolding process requires overcoming the hydrophobic effect by hydrating the amino acids or achieving the melting temperature of the protein. The heat of hydration is a destabilization of a protein. Typically, the folded state of a protein is favored by only 5-15 kcal/mole over the unfolded state. Nonetheless, protein unfolding at neutral pH and at room temperature requires chemical reagents. In fact, partial unfolding of a protein is often observed prior to the onset of irreversible chemical or conformation processes. Moreover, protein conformation generally controls the rate and extent of deleterious chemical reactions.

Conformational protection of active agents by proteins depends on the stability of the protein's folded state and the thermodynamics associated with the agent's decomposition. Conditions necessary for the agent's decomposition should be different than for protein unfolding.

Variable molecular weights of the carrier compound can have profound effects on the active agent release kinetics. As a result, low molecular weight active agent delivery systems are preferred. An advantage of this invention is that chain length and molecular weight of the polypeptide can be optimized depending on the level of conformational protection desired. This property can be optimized in concert with the kinetics of the first order release mechanism. Thus, another advantage of this invention is that prolonged release time can be imparted by increasing the molecular weight of the carrier polypeptide. Another, significant advantage of the invention is that the kinetics of active agent release is primarily controlled by the enzymatic hydrolysis of the key bond between the carrier peptide and the active agent.

Dextran is the only polysaccharide known that has been explored as a macromolecular carrier for the covalent binding of drug for colon specific drug delivery. Generally, it was only possible to load up to 1/10 of the total drug-dextran conjugate weight with drug. As stated earlier, polysaccharides are digested mainly in the colon and drug absorption is mainly limited to the colon. As compared to dextran, this invention has two major advantages. First, peptides are hydrolyzed by any one of several aminopeptidases found in the intestinal lumen or associated with the brush-border membrane and so active agent release and subsequent absorption can occur in the jejunum or the ileum. Second, the molecular weight of the carrier molecule can be controlled and, thus, active agent loading can also be controlled.

The alcohol group of the active agent is covalently attached to the N-terminus of the oligopeptide or polypeptide.

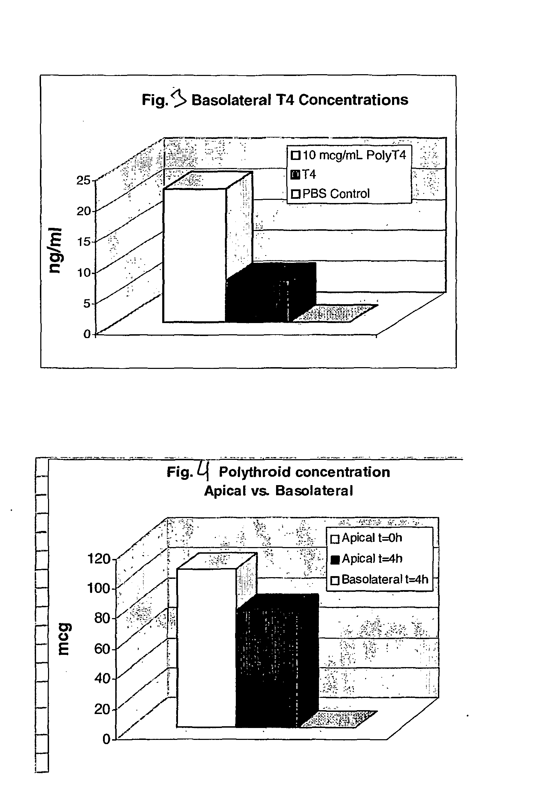

In the N-terminus example; the peptide is, in essence, extended by one monomeric unit forming a new peptide bond. When the active agent is an alcohol; the N-terminus is the preferred point of attachment in order to achieve a stable composition. An alcohol can be converted into an alkylchloroformate with phosgene. This invention, then, pertains to the reaction of this key intermediate with the N-terminus of the peptide carrier as shown in Fig. 3. Fig. 3 also depicts the release of the active ingredient from the peptide carrier by intestinal peptidases.

The polypeptide carrier can be prepared using conventional techniques.

The addition of stabilizers to the composition has the potential of stabilizing the polypeptide further. Stabilizers such as sugar, amino acids, polyethylene glycol (PEG) and salts have been shown to prevent protein unfolding. In another embodiment of the invention, a pre-first order release of the active agent is imparted by microencapsulating the carrier polypeptide-active agent conjugate in a polysaccharide, amino acid complex, PEG or salts.

There is evidence that hydrophilic compounds are absorbed through the intestinal epithelia efficiently via specialized transporters. The entire membrane transport system is intrinsically asymmetric and responds asymmetrically to cofactors. Thus, one can expect that excitation of the membrane transport system will involve some sort of specialized adjuvant resulting in localized delivery of active agents. There are seven known intestinal transport systems classified according to the physical properties of the transported substrate. They include the amino acid, oligopeptide, glucose, monocarboxic acid, phosphate, bile acid and the P-glycoprotein transport systems and each has its own associated mechanism of transport. The mechanisms can depend on hydrogen ions, sodium ions, binding sites or other cofactors. The invention also allows targeting the mechanisms for intestinal epithelial transport systems to facilitate absorption of active agents.

In another embodiment of the invention, the composition includes one or more adjuvants to enhance the bioavailability of the active agent. Addition of an adjuvant is particularly preferred when using an otherwise poorly absorbed active agent. Suitable adjuvants, for example, include: papain, which is a potent enzyme for releasing the catalytic domain of aminopeptidase-N into the lumen; glycorecognizers, which activate enzymes in the BBM; and bile acids, which have been attached to peptides to enhance absorption of the peptides.

Preferably, the resultant peptide-active agent conjugate is formulated into a tablet using suitable excipients and can either be wet granulated or dry compressed.

Compositions of the invention are, in essence, the formation of amides from acids and amines and can be prepared by the following examples.

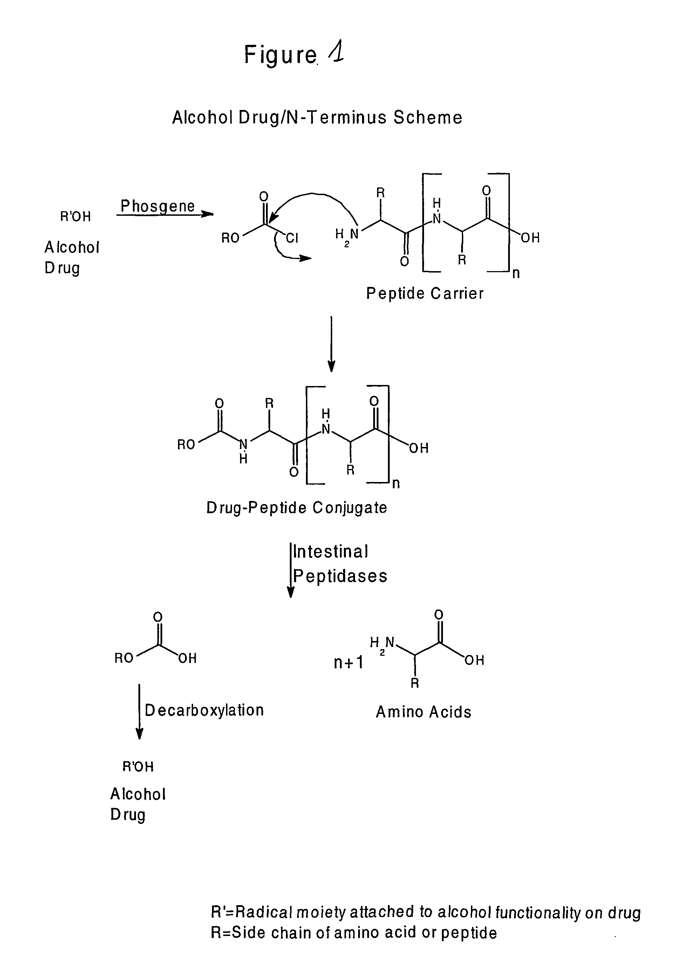

### Alcohol/N-Terminus Conjugation (Fig. 1)

In the following example the combination of the alcohol with triphosgene produces a chloroformate, which when reacted with the N-terminus of the peptide produces a carbamate. Pursuant to this, an alcohol bioactive agent can be treated with triphosgene in dry DMF under nitrogen. The suitably protected peptide carrier is then added slowly and the solution stirred at room temperature for several hours. The product is then precipitated out in ether The crude product is suitably deprotected and purified using GPC.

Other solvents, activating agents, cocatalysts and bases can be used. Examples of other solvents include dimethylsulfoxide, ethers such as tetrahydrofuran or chlorinated solvents such as chloroform. Examples of other activating agents include dicyclohexylcarbodiimide or thionyl chloride. An example of another cocatalyst is N-hydroxysuccinimide. Examples of bases include pyrrolidinopyridine, dimethylaminopyridine, triethylamine or tributylamine.

### EXAMPLE 1

### Preparation of Capped Iodothyronine Composition Comprising a Copolymer of T₃ and T₄ Covalently Attached to the N-terminus of Polyglutamic Acid

The synthesis of polyglutamic acid is well known through a variety of reported methods. For the present examples polyglutamic acid was synthesized through the activation of the Benzyl Glutamic NCA (BnGlu-NCA) monomer. The BnGlu-NCA was then polymerized and the benzyl groups removed with hydrogen bromide. When capping polyglutamic acid, the liberation of its N-terminus amino group from the hydrogen bromide complex without imparting unwanted nucleophilicity to the free carboxylic acids is critical. Reactions using sodium carbonate, sodium bicarbonate, and sodium acetate produced glutamic acid/T₄/T₃ copolymer with the T₄ and T₃ incorporation decreasing with increasing pKb. Sodium acetate was the preferred reagent because its pKb is between that of sodium bromide, polyglutamic acid, and sodium salt. The reaction using basic alumina kept the T₄-NCA and T₃-NCA intact with no apparent capping or self-polymerization. The stability of T₄-NCA and T₃-NCA will influence how glutamic acid/T₄/T₃ copolymer will be commercially manufactured. Sodium acetate can be replaced with sodium carbonate, sodium bicarbonate, sodium propionate, sodium butyrate, sodium pivalate, basic alumina, or any other weak base capable of neutralizing hydrogen bromide complexed with an amino group.

The synthesis of glutamic acid/T₄/T₃ copolymer began with benzylglutamic acid, thyroxine, and triiodothyronine. Each of these synthons was independently reacted with triphosgene in a suitable organic solvent. The BnGlu-NCA was polymerized in tetrahydrofuran (THF) with sodium methoxide as an initiator. Polybenzylglutamic acid was deprotected with 15% hydrogen bromide in acetic acid. This product needs to be free of uncomplexed hydrogen bromide where it was dissolved in DMF and treated with sodium acetate. The previously prepared T₄-NCA and T₃-NCA were blended and added to the solution. The reaction was then stirred until T₄-NCA or T₃-NCA were no longer detected by thin layer chromatography (TLC). The final product was added to water and the precipitate was washed with water and dried *in vacuo* to yield a white amorphous powder.

Experimentation with several weak bases revealed that a variety of sodium salts of a carboxylic acid work in capping polyglutamic acid. The reaction was tried with sodium propionate, sodium butyrate, and sodium pivalate in lieu of sodium acetate all with essentially the same result.

### Preparation of benzylglutamic acid-NCA

Benzylglutamic acid (25 grams) was suspended in 400 mL anhydrous ethyl acetate under nitrogen. The mixture was heated to reflux where 30 grams of triphosgene was added in six (6) equal portions. The reaction was refluxed for three (3) hours until homogenous. The solution was cooled to room temperature, filtered, and concentrated *in vacuo.* The white powder was recrystallized from 50 mL of hot anhydrous ethyl acetate to yield 17.4 grams (63%) of a white powder.

### Preparation of T₄-NCA

In a round bottom flask fitted with a nitrogen inlet, five grams of thyroxine was stirred with 25 mL of tetrahydrofuran (THF) and 1.3 grams of triphosgene and the mixture refluxed for four (4) hours until homogenous. The solution was cooled to room temperature, and added dropwise to 200 mL of heptane with stirring. The crystals were filtered and dried *in vacuo* to yield 4.72 grams (91%) of an off-white powder.

### Preparation of T₃-NCA

In a round bottom flask fitted with a nitrogen inlet, 4.29 grams of triiodothyronine was stirred with 20 mL of tetrahydrofuran (THF) and 1.45 grams of triphosgene and the mixture refluxed for four (4) hours until homogenous. The solution was cooled to room temperature and added dropwise to 200 ml of heptane with stirring. The liquid was decanted off the yellow gum, which was recrystallized, from anhydrous ethyl acetate and hexanes to yield 2.5 grams (56%) of a white powder that was dried under high vacuum.

### Preparation of polybenzylglutamic acid

Benzylglutamic acid (17.4 grams) was dissolved in anhydrous tetrahydrofuran (THF) under nitrogen where 238 mg of sodium methoxide was added portionwise. The solution was stirred for two (2) days with a marked increase in viscosity. The solution was poured into 1.5 L of petroleum ether with stirring. The petroleum ether was decanted off and an additional 1L of petroleum ether was added back. The mixture was stirred by hand, the petroleum ether was decanted off and the process repeated with 500 mL of petroleum ether. The white solid was air dried and then vacuum dried to yield 14.7 (95%) of a white fluffy paper-like solid.

### Preparation of polyglutamic acid

Acetic acid (10mL) was stirred with 0 mL 30wt% hydrogen bromide (HBr) in acetic acid where 1.96 of polybenzylglutamic acid was added by hand. The mixture was stirred at room temperature for one day and was, then, added to 50 mL of ether. The white precipitant was filtered, washed with 4 x 30 mL of ether and dried under a high vacuum to yield 1.11 grams (97%) of a white powder.

### Preparation of glutamic acid/T₄/T₃ copolymer

Polyglutamic acid (375 mg) was dissolved in dry 3 mL DMF. Sodium acetate (24 mg) was added followed by a blend of 105 mg of T₄-NCA and 8 mg of T₃-NCA. The solution was stirred for two (2) days where TLC showed the absence of thyronine starting materials. The solution was poured into 30 mL of water and cooled 10°C overnight. The precipitant was filtered, washed with water, and dried under high vacuum to yield 413 mg (85%) of light beige powder. The proton NMR revealed a copolymer of T₃ and T₄ covalently attached to the N-terminus of polyglutamic acid, which was virtually completely digested by the pronase enzyme system in two hours.

### EXAMPLE 2

Monolayers of Caco-2 human intestinal epithelial cells are increasingly being used to predict the absorption of orally delivered drugs. We used the Caco-2 transwell system and other *in vitro* assays to evaluate the performance of Polythroid. Our findings indicate that Polythroid may enhance oral delivery of thyroid hormones for the treatment of hypothyroid disorders.

### IN VITRO PERFORMANCE

### Caco-2 human intestinal epithelial cell assay

Caco-2 cells are grown on the surface of collagen coated wells in a 24 well format to form confluent monolayers that represent small segments of the intestine. The wells are removable and contain a top chamber representing the apical side (facing the lumen of the intestine) and a bottom chamber representing the basolateral side (site of serosal drug absorption). The integrity of the epithelial barrier is monitored by testing the electrical resistance across the monolayer. Absorption of drugs can be studied by adding sample to the apical side and assaying the concentration of the drug in the basolateral chamber following incubation.

### Intestinal epithelial cell proteases digest Polythroid

Polythroid is a synthetic polymer of glutamic acid with T4 and T3 covalently attached by a peptide bond linkage. The polymer is the delivery vehicle for the thyroid hormones and is not designed to cross the intestinal barrier itself. Rather, it is designed to release T4 and T3 in a time dependent manner. Release of the thyroid hormones is dependent on the enzymatic cleavage of the glutamic acid polymer. In theory, this will result from Polythroid encountering proteolytic enzymes as it descends the intestinal tract. Proteins are digested into small polypeptides by gastric pepsin and pancreatic enzymes secreted into the small intestine. Intestinal epithelial cells then function to further breakdown the small polypeptides. They accomplish this with proteolytic enzymes referred to as brush border proteases that are attached to the cell surface.

Monitoring the effect of brush border peptidases on Polythroid required development of an assay to specifically distinguish Polythroid from polyglutamic acid and the thyroid hormones. Therefore, we developed an enzyme-linked immunosorbent assay (ELISA) that specifically recognizes Polythroid. The assay employs antibodies against the glutamic acid polymer to capture Polythroid and antibodies to T4 or T3 to detect the presence of Polythroid. The assay has no cross-reactivity with polyglutamic acid or the thyroid hormones themselves. Consequently, proteolytic degradation of Polythroid results in T4 and T3 release from the polymer and a corresponding decrease in ELISA reactivity. The Polythroid specific ELISA can, therefore, be used to monitor the breakdown of Polythroid.

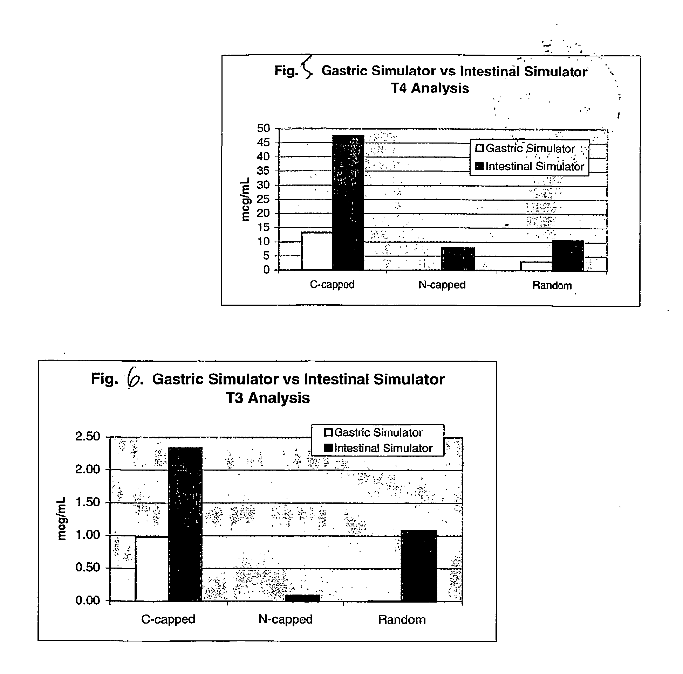

The Polythroid specific assay was used to analyze *in situ* digestion of Polythroid in Caco-2 cell cultures. Different concentrations of Polythroid were added to the apical side of Caco-2 cells and incubated for 4 hours in PBS at 37°C (n=4). The apical side Polythroid concentration was measured by Polythroid specific ELISA before and after the 4 hour incubation (Fig. 6). At the relatively high concentration of 100 micrograms, 26% of Polythroid was degraded, whereas at a 10-fold lower concentration 84% of the Polythroid was degraded. When a concentration of 0.5 micrograms was added (closer to the concentrations that would be encountered by the intestine in a normal human dose) the amount of Polythroid remaining after 4 hours of incubation was below the limit of detection for the ELISA (10 ng) indicating essentially complete digestion. The loss of Polymer in the apical chamber was not due to absorption of Polythroid across the monolayer since the basolateral chamber contained no detectable Polythroid in any of the experiments (see below). We cannot rule out cellular uptake of Polythroid, however, enzymatic digestion is likely to account for most, if not all, of the decrease in Polythroid concentration on the apical side. At the higher concentrations, it would be difficult for cellular uptake to account for such a large difference in the remaining Polythroid.

### Polythroid enhances absorption of T4 across Caco-2 monolayers

Absorption of T4 was monitored in the Caco-2 transwell system (n=4). Polythroid (10 micrograms) was added to the apical side of the transwells. T4 was added to the apical side at a concentration equal to the T4 content of Polythroid. A commercial ELISA for T4 was used to determine the level of T4 in the basolateral chamber following incubation for 4 hours at 37°C (Fig. 7). A significantly higher amount of T4 was absorbed from Polythroid as compared to CaCo-2 cells incubated with the amount of T4 equivalent to that contained in the polymer.

### Polythroid does not cross Caco-2 monolayers

In order to determine if Polythroid itself crosses the Caco-2 monolayer we used the Polythroid specific ELISA to measure the amount of polymer in the basolateral chamber after incubation with Polythroid at a high concentration (100 micrograms). After 4 hours incubation, samples (n=4) from the basolateral side showed no reactivity in the ELISA (Fig. 8). The limit of detection for Polythroid is 10 ng, therefore, less than 1/10,000 of the Polythroid was absorbed. In conclusion, within the limits of ELISA detection, Polythroid does not cross the Caco-2 monolayer.

### Digestion of Polythroid in gastric and intestinal simulators

Pepsin secreted by the gastric mucosa is the only protease active in the acid conditions of the stomach. The pancreas secretes a number of proteolytic enzymes into the intestine which degrade proteins and polypeptides. In theory, these endogenous proteases will participate in release of T4 and T3 from Polythroid as the polymer descends the intestinal tract.

We tested Polythroid in the USP gastric simulator and the USP intestinal simulator and compared the levels of digestion for Polythroid synthesized by different methods. The samples of Polythroid varied in the position of thyroid hormone attachment. Samples were dissolved in gastric simulator buffer containing pepsin or in intestinal simulator buffer containing pancreatic enzyme extract (pancreatin) and incubated for 24 hours at 37°C. Following digestion, samples were analyzed by HPLC for the content of released monomeric T4 and T3. Figures 9and 10 show the levels of T4 and T3 following digestion in the gastric and intestinal simulators. Release varied depending on the position of thyroid hormone attachment. Polythroid with T4 and T3 attached at the C-terminus (C-capped) showed the highest level of digestion. On the other hand, Polythroid with N-terminal attachment (N-capped) showed no digestion in the gastric simulator and a relatively low amount of digestion in the intestinal simulator. Polythroid with random attachment showed only marginal digestion in the gastric simulator and moderate digestion in the intestinal simulator. In conclusion, the rate of thyroid hormone release from Polythroid varies depending on the method of synthesis. This provides a potential means of controlling (fine tuning) time release of oral delivery.

### Conclusions and Summary

The following conclusions can be drawn from *in vitro* performance assays:
- T4 and T3 are released from Polythroid by pancreatic and intestinal cell proteases
- T4 and T3 released from Polythroid are absorbed across intestinal monolayers
- Polythroid enhances absorption of T4 across intestinal epithelium *in vitro*
- Polythroid itself does not cross the intestinal epithelial barrier *in vitro*
- The kinetics of time release may be controlled by the method of Polythroid synthesis

Covalent attachment of T4 and T3 to a polypeptide affords a number of potential advantages to oral delivery for thyroid hormone replacement therapy. Proteolytic enzymes produced by the pancreas and intestinal epithelial cells release T4 and T3 from Polythroid. Therefore, T4 and T3 should be released in a time dependent manner as they descend the intestinal tract. Once released the hormones are absorbed across the intestinal epithelium in the Caco-2 cell model. In addition, data from the *in vitro* intestinal epithelial model suggests that attachment of T4 to polymers of glutamic acid may enhance absorption of the thyroid hormones, perhaps by providing a second mechanism of uptake and/or enhancing solubility of the hormones. Polythroid itself does not cross the intestinal epithelial barrier in the *in vitro* Caco-2 model. Thus, any concerns about systemic effects of the polymer are minimized since it should not be absorbed into the bloodstream.

## Claims

1. A drug-peptide conjugate comprising
• a polypeptide consisting of polyglutamic acid, and
• a copolymer of T3 and T4 covalently attached through an alcohol functionality of said copolymer to the N-terminus of said polypeptide.

2. A composition comprising a drug-peptide conjugate according to claim 1, wherein said composition is in a form suitable for oral administration and enzymatic release of T3 and T4 following oral administration.

3. A pharmaceutical composition comprising a drug-peptide conjugate according to claims 1 or 2.

4. A method for controlling the release of T3 and T4 from a composition comprising a drug-peptide conjugate according to claims 1 or 2, **characterised in that** said T3 and T4 are released from said composition by an enzyme-catalyzed release.

5. Use of a drug-peptide conjugate according to claims 1 or 2 for preparing a medicament useful for treating hypothyroid disorders.

## Patentansprüche

1. Arzneimittel-Peptidkonjugat, das Folgendes aufweist:
• ein Polypeptid, bestehend aus Polyglutaminsäure, und
• ein Copolymer aus T3 und T4, das über eine Alkoholfunktion des Copolymers an den N-terminus des Polypeptids gebunden ist.

2. Zusammensetzung, die ein Arzneimittel-Peptidkonjugat nach Anspruch 1 aufweist, wobei die Zusammensetzung in einer Form vorliegt, die zur oralen Verabreichung und zur enzymatischen Freisetzung von T3 und T4 im Anschluss an die orale Verabreichung geeignet ist.

3. Pharmazeutische Zusammensetzung, die ein Arzneimittel-Peptidkonjugat nach Anspruch 1 oder 2 aufweist.

4. Verfahren zur Kontrolle der Freisetzung von T3 und T4 aus einer Zusammensetzung, die ein Arzneimittel-Peptidkonjugat nach Anspruch 1 oder 2 aufweist, **dadurch gekennzeichnet, dass** das T3 und T4 durch eine enzymkatalysierte Freisetzung aus der Zusammensetzung freigesetzt werden.

5. Verwendung eines Arzneimittel-Peptidkonjugats nach Anspruch 1 oder 2, zur Herstellung eines Medikaments, das zur Behandlung von hypothyroiden Störungen nützlich ist.

## Revendications

1. Conjugué médicament-peptide comprenant :
■ un polypeptide constitué d'acide polyglutamique, et
■ un copolymère de T3 et T4 fixé de façon covalente par une fonctionnalité alcool dudit copolymère à l'extrémité N-terminale dudit polypeptide.

2. Composition comprenant un conjugué médicament-peptide selon la revendication 1, où ladite composition se présente sous une forme convenant à l'administration orale et à la libération enzymatique de T3 et T4 après administration orale.

3. Composition pharmaceutique comprenant un conjugué médicament-peptide selon la revendication 1 ou 2.

4. Procédé de contrôle de la libération de T3 et T4 d'une composition comprenant un conjugué médicament-peptide selon la revendication 1 ou 2, **caractérisé en ce que** lesdites T3 et T4 sont libérées de ladite composition par libération catalysée enzymatiquement.

5. Utilisation d'un conjugué médicament-peptide selon la revendication 1 ou 2 pour préparer un médicament utile pour traiter les troubles hypothyroïdiens.
